# EUROPÄISCHE PATENTSCHRIFT

(11) **EP 1 002 783 B1**
(45) Veröffentlichungstag und Bekanntmachung des Hinweises auf die Patenterteilung: **23.04.2003**
(21) Anmeldenummer: 99121810.8
(22) Anmeldetag: 04.11.1999
(51) Int. Cl.: C07C 43/32, C07C 41/60

(54) **Verfahren zur Herstellung von Orthoestern**
Process for the preparation of ortho esters
Procédé pour la préparation d'ortho esters

(30) Priorität: 18.11.1998 DE 19853089
(43) Veröffentlichungstag der Anmeldung: 24.05.2000
(73) Patentinhaber: BASF AKTIENGESELLSCHAFT, 67056 Ludwigshafen (DE)
(72) Erfinder: Burkart, Kirsten, 67069 Ludwigshafen (DE); Guth, Josef, 67251 Freinsheim (DE); Letzelter, Thomas, 76855 Annweiler (DE); Schweinzer, Jürgen, Dr., 67227 Frankenthal (DE); Sterzel, Hans-Josef, Dr., 67125 Dannstadt-Schauernheim (DE)

(56) Entgegenhaltungen:
- DE-A- 2 104 206
- DE-A- 3 606 472
- DE-B- 1 217 943
- DE-C- 919 465
- "Organic Syntheses, Collective Volume I, Second Edition" 1946 , WILEY , NEW YORK XP002131065 * Seite 258 - Seite 261 *

## Beschreibung

Die vorliegende Erfindung betrifft ein Verfahren zur Herstellung von Orthoestern durch Umsetzung von Metallalkoholaten mit Verbindungen der Formel R'-CHal₃. Orthoester, insbesondere die Trialkylorthoester und darunter besonders die Trialkylorthoester der Ameisensäure ("Orthoformiate"), sind wichtige Synthesebausteine in der organischen Chemie und werden beispielsweise zur Herstellung von meist komplexen organischen Strukturen wie Pharmazeutika oder Pflanzenschutzmitteln verwendet. Die wirtschaftlich bedeutendsten Orthoester sind die beiden Orthoformiate Trimethylorthoformiat ("TMOF") und Triethylorthoformiat ("TEOF"),

Verfahren zur Herstellung von Orthoestern aus Metallalkoholaten und Verbindungen der Formel R'-CHal₃ sind seit langem bekannt. Üblicherweise werden dazu drei Mole eines Metallalkoholats MOR (M = Metall, R = organischer Rest) mit einer Verbindung der Formel R'-CHal₃ (Hal = F, Cl, Br, I, R' = H oder organischer Rest) umgesetzt, wobei sich unter Abscheidung von Alkalihalogenid der Orthoester R'-C(OR)₃ bildet. Zur Herstellung von Orthoformiaten wird dementsprechend Alkalialkoholat MOR mit Trihalogenmethan H-CHal₃ umgesetzt. Zumeist wird bei der Herstellung von Orthoestern als Halogen Chlor verwendet und als Alkalimetall Natrium, da dies die billigsten Vertreter ihrer Gruppen sind. Zur technischen Herstellung von TMOF oder TEOF wird demgemäß üblicherweise Natriummethanolat (M = Na, R = CH₃) oder Natriumethanolat (M = Na, R = CH₂CH₃) mit Chloroform (Trichlormethan, R' = H, Hal = Cl) umgesetzt.

Meist werden die Verfahren zur Herstellung von Orthoestern in homogener Lösung (absehen vom ausfallenden Alkalihalogenid) mit dem entsprechenden Alkohol als Lösungsmittel durchgeführt; TMOF wird folglich in methanolischer Lösung und TEOF in ethanolischer Lösung hergestellt. Häufig wird dabei das Alkalialkoholat durch Verwendung einer Lösung von Alkalihydroxid im Alkohol erst vor oder bei der Umsetzung im Gleichgewicht gebildet. So lehrt DE-A 21 04 206 ein Verfahren zur kontinuierlichen Herstellung von Trialkylorthoformiaten durch Umsetzung von Chloroform mit einer Lösung eines Alkalialkoholats im entsprechenden Alkohol, wobei die Umsetzung in Abwesenheit von Wasser und Sauerstoff bei Temperaturen zwischen 40 und 120 °C und einem Druck von 1 bis 8 bar durchgeführt wird. PL-B 125872 offenbart die Herstellung von Triethylorthoformiat durch Umsetzung von Chloroform mit drei Molen Natriumethanolat in Ethanol. CN-A 1106375 offenbart die Herstellung von Triethylorthoformiat durch die langsame Zugabe von Ethanol zu einem Gemisch aus Natriumhydroxid und Chloroform, wobei der pH des Reaktionsgemisches zwischen 7 und 10 gehalten wird. JP-A 59-001 435 lehrt die Herstellung von Trialkylorthoformiaten durch Umsetzung des Alkohols, einer Lösung von 5 bis 23 Gew.-% Alkalihydroxid im Alkohol, und Chloroform. DE-A 36 06 472 lehrt ein Verfahren zur Herstellung von Trialkylorthoformiaten durch isotherme Umsetzung von Chloroform und einer alkoholischen Lösung von Alkalialkoholat bei 1 bis 6 bar Druck, einer Temperatur von 30 bis 120 °C und einem pH-Wert oberhalb von 7, wobei ein stöchiometrischer Überschuß von Chloroform angewendet wird. Das molare Verhältnis von Chloroform zum Alkoholat beträgt 1 bis 2 zu 3.

Diese Verfahren weisen den Nachteil auf, daß das alkoholische Lösungsmittel vom Orthoester abgetrennt werden muß. Bei der Gewinnung des Orthoesters aus üblichen Reaktionsgemischen sind dabei zur Erzielung der vom Markt geforderten Produktreinheit von Orthoestern Destillationskolonnen mit hohen Trennstufenzahlen und ein entsprechend hoher Energieaufwand erforderlich.

Daher wurde mit anderen Verfahren versucht, die Verwendung von Alkohol als Lösungsmittel oder zumindest die Destillation von Orthoester/Alkohol-Gemischen zu vermeiden.

So lehrt DE-A 12 17 943 die Umsetzung von Chloroform mit einem im herzustellenden Trialkylorthoformiat suspendierten Metallmethanolat, wobei eine Ausbeute von 84 Mol-%, bezogen auf eingesetztes Methanolat, erreicht wird. JP-A 58-225 036 lehrt die Extraktion von Orthoestern aus einem Gemisch von Alkohol, Alkalihydroxid und Chloroform mit wahlweise halogensubstituierten aliphatischen oder aromatischen Kohlenwasserstoffen oder Ethern. CN-A 10 68 103 lehrt die Verwendung eines inerten Lösungsmittels bei der Umsetzung.

Andere Verfahren werden in Gegenwart eines Phasentransferkatalysators durchgeführt. RU-A 20 72 978 und SU-A 1781203 lehren die Umsetzung eines Alkanols und eines Alkalimetallhydroxids mit Chloroform in Gegenwart von Phasentransferkatalysatoren.

Diesen bekannten Verfahren mit heterogenen Reaktionsgemischen (das ausfallende Alkalihalogenid nicht gerechnet) ist jedoch der Nachteil der Bildung von Nebenprodukten in vergleichsweise hoher Menge zu eigen; die Selektivität der Reaktion ist unbefriedigend. Die Extraktionsverfahren weisen neben meist unbefriedigender Selektivität der Reaktion den Nachteil einer zusätzlichen Verfahrensstufe auf.

SU-A 16 71 656 lehrt ein weiteres Verfahren zur Herstellung von Orthoestern durch Umsetzung von Chloroform mit dem entsprechenden Tetraalkoxisilan in Pseudocumol als Lösungsmittel in Gegenwart von Alkalihydroxid und Triethylbenzylammoniumchlorid. Dieses Verfahren ist wegen der Verwendung vergleichsweise kostspieliger Chemikalien wirtschaftlich unbefriedigend.

Es bestand daher die Aufgabe, ein wirtschaftlich befriedigendes Verfahren zur Herstellung von Orthoestern mit hoher Selektivität, hoher Raumzeitausbeute und niedrigem Produktreinigungsaufwand zu finden.

Dementsprechend wurde ein Verfahren zur Herstellung eines Orthoesters der Formel R'-C(OR)₃, wobei R und R' jeweils einen organischen Rest und R' auch Wasserstoff darstellen und wobei die Reste R im Orthoester gleich oder voneinander verschieden sind, durch Umsetzung einer Verbindung der Formel R'-CHal₃, wobei Hal Halogen darstellt, mit einem Alkalialkoholat der Formel ROM, wobei M Alkali darstellt, in Gegenwart des entsprechenden Alkohols der Formel ROH, oder im Falle voneinander verschiedener Reste R im Orthoester mit einem entsprechenden Gemisch von Alkalialkoholaten in einem entsprechenden Gemisch von Alkoholen gefunden, das dadurch gekennzeichnet ist, daß man eine Maische des Alkalialkoholats oder der Alkalialkoholate im entsprechenden Alkohol oder Gemisch von Alkoholen einsetzt.

Mit dem erfindungsgemäßen Verfahren wird die Abtrennung hoher Alkoholmengen vermieden, der Aufwand für die Gewinnung des reinen Produkts ist also wesentlich niedriger als mit bekannten Verfahren. Gleichzeitig tritt trotz des Vorhandenseins von festem Alkalialkoholat im Reaktionsmedium überraschenderweise keine Selektivitätsverschlechterung gegenüber der Verwendung einer reinen Lösung des Alkoholats im Alkohol auf.

Mit dem erfindungsgemäßen Verfahren werden Orthoester der Formel R'-C(OR)₃ durch Umsetzung eines Alkalialkoholats MOR mit einer Verbindung der Formel R'-CHal₃ hergestellt. Es ist mit dem erfindungsgemäßen Verfahren ebenso möglich, gemischte Orthoester herzustellen, also solche, die nicht drei gleiche Reste R, sondern zwei oder drei unterschiedliche Reste tragen, also Orthoester der Formeln R'-C(OR¹)2(OR²) oder R'-C(OR¹)(OR²)(OR³). R¹, R² und R³ entsprechen dabei unabhängig voneinander je einem Rest R. Zu ihrer Herstellung sind die entsprechenden Alkoholate MOR¹, MOR² und/oder MOR³ sowie die entsprechenden Alkohole HOR¹, HOR² und/oder HOR³ im gewünschten Verhältnis einzusetzen.

M steht für Alkalimetall, also Lithium, Natrium, Kalium, Rubidium und/oder Cäsium. Es ist möglich, ein einzelnes Alkalimetall oder ein Gemisch verschiedener Alkalimetalle einzusetzen. Bevorzugt ist die Verwendung von Natrium oder Kalium, besonders bevorzugt ist die Verwendung von Natrium.

Hal steht für Halogen, also Fluor, Chlor, Brom und/oder Iod. Es kann ein einzelnes Halogen oder ein Gemisch von Halogenen verwendet werden. Bevorzugt ist die Verwendung von Chlor.

R steht für einen organischen Rest, wie Alkyl, Alkenyl, Alkinyl, Cycloalkyl, Cycloalkenyl, Aryl, Alkaryl oder Aralkyl. Der Rest R kann mit weiteren organischen Resten substituiert sein und auch Heteroatome umfassen.

Beispiele für im erfindungsgemäßen Verfahren verwendbare Reste R sind lineare gesättigte Alkylreste mit einem bis 18 Kohlenstoffatomen wie Methyl, Ethyl, Propyl, Butyl, Pentyl, Hexyl, Heptyl, Octyl, Nonyl oder Decyl, gesättigte cyclische Alkylreste mit drei bis 12 Kohlenstoffatomen wie Cyclopentyl, Cyclohexyl oder Cycloheptyl, oder verzweigte gesättigte Alkylreste wie 2-Propyl, 2-Butyl, 2-Methyl- 1-propyl, 1,1-Dimethylethyl oder alle verzweigten isomeren Pentyl-, Hexyl-, Heptyl-, Octyl-, Nonyl- oder Decylreste. R kann ebenso ein aromatischer Rest sein, beispielsweise Phenyl oder 1- oder 2-Naphthyl. Vorzugsweise ist R ein linearer Alkylrest, insbesondere ein Methyl- Ethyl-, 1-Propyl- oder 1-Butylrest. In besonders bevorzugter Weise ist R ein Methyl- oder ein Ethylrest.

R' steht für einen Wasserstoffrest oder einen organischen Rest R wie oben definiert.

Beispiele für im erfindungsgemäßen Verfahren verwendbare organische Reste R' sind lineare gesättigte Alkylreste mit einem bis 18 Kohlenstoffatomen wie Methyl, Ethyl, Propyl, Butyl, Pentyl, Hexyl, Heptyl, Octyl, Nonyl oder Decyl, gesättigte cyclische Alkylreste mit drei bis 12 Kohlenstoffatomen wie Cyclopentyl, Cyclohexyl oder Cycloheptyl, oder verzweigte gesättigte Alkylreste wie 2-Propyl, 2-Butyl, 2-Methyl- 1-propyl, 1,1-Dimethylethyl oder alle verzweigten isomeren Pentyl-, Hexyl-, Heptyl-, Octyl-, Nonyl- oder Decylreste. R' kann ebenso ein aromatischer Rest sein, beispielsweise Phenyl oder 1- oder 2-Naphthyl (das 1,1,1-Trihalogenalkan ist dann ein aromatisch substituiertes 1,1,1-Trihalogenmethan). Vorzugsweise ist R' ein Wasserstoff- oder ein linearer Alkylrest, insbesondere ein Methyl- Ethyl-, 1-Propyl- oder 1-Butylrest. In besonders bevorzugter Weise ist R' ein Wasserstoff-, ein Methyl- oder ein Ethylrest.

In den besonders bevorzugten Ausführungsformen des erfindungsgemäßen Verfahrens wird entweder durch Umsetzung von Natriummethanolat (R = Methyl, M = Na) mit Chloroform (R' = H, Hal = Cl) TMOF oder durch Umsetzung von Natriumethanolat (R = Ethyl, M = Na) mit Chloroform (R' = H, Hal = Cl) TEOF hergestellt.

Die Alkalialkoholate werden auf bekannte Weise hergestellt, beispielsweise durch Umsetzung von Alkalihydroxid mit dem Alkohol, Auflösen des Alkalimetalls im entsprechenden Alkohol oder Zersetzung des im Amalgamverfahren zur Chloralkalielektrolyse gebildeten Alkaliamalgams mit dem entsprechenden Alkohol. Die Alkalialkoholate sind als handelsübliche Waren auch kommerziell erhältlich.

Das Alkalialkoholat MO-R wird im erfindungsgemäßen Verfahren mit dem entsprechenden Alkohol, also dem Alkohol HO-R, der durch den formalen Ersatz des Alkaliions im Alkalialkoholat MO-R durch ein Proton hervorgeht, angemaischt.

Die Verbindung der Formel R'-CHal₃ wird mit einer Maische des Alkalialkoholats im entsprechenden Alkohol umgesetzt. Unter "Maische" ist eine Aufschlämmung des festen Alkalialkoholats im Alkohol zu verstehen, wobei sich lediglich der in der angewandten Alkoholmenge lösliche Teil des Alkalialkoholats im Alkohol löst und der Rest ungelöst bleibt. Die Maische ist folglich eine Aufschlämmung eines Alkalialkoholats in einer gesättigten Lösung des Alkalialkoholats im entsprechenden Alkohol. Der Feststoffgehalt der Maische wird so eingestellt, daß die Viskosität der Maische technisch beherrschbar ist, die Maische in den verwendeten Reaktoren also durchmischbar und - wenn gewünscht oder erforderlich - pumpfähig ist, aber der Alkoholgehalt möglichst niedrig ist. Zu hohe Temperaturspitzen an den Stellen, an denen Reaktanten in den Reaktor eingeführt werden, müssen vermieden werden, da dadurch die Selektivität der Reaktion sinkt. Die Obergrenze für die Viskosität der gesamten Reaktionsmischung ergibt sich daher aus der Fähigkeit des verwendeten Reaktors, die Komponenten der Reaktionsmischung ausreichend schnell zu durchmischen.

Im allgemeinen wird eine Maische mit einem Gewichtsverhältnis von festem zu gelöstem Alkalialkoholat von mindestens 0,4 verwendet, bevorzugterweise beträgt dieses Verhältnis mindestens 0,7 und in besonders bevorzugter Weise mindestens 0,9. Das Gewichtsverhältnis beträgt im allgemeinen höchstens 3, bevorzugterweise höchstens 2 und in besonders bevorzugter Weise höchstens 1,5.

Zur Durchführung des erfindungsgemäßen Verfahrens geeignete Reaktoren sind dem Fachmann bekannt. Es wird ein Reaktor verwendet, der in der Lage ist, die viskose Reaktionsmischung zu durchmischen. Bevorzugt sind Reaktoren, die Feststoffe oder eine Maische mit hohem Feststoffanteil schnell mit flüssigen Komponenten durchmischen können, beispielsweise Schaufeltrockner, Kneter oder Discotherm-Reaktoren. Eine weitere Anforderung an den Reaktor ist, das am Ende der Reaktion vorliegende Alkalihalogenid gut trocknen zu können, also die flüchtigen Komponenten der Reakti-onsmischung möglichst vollständig entfernen zu können. Der Reaktor ist daher vorzugsweise heizbar. Weiterhin muß der Reaktor über einen Kühler, beispielsweise einen angeschlossenen Siedekühler, einen außen- oder einen innenliegenden Wärmetauscher verfügen, um die bei der Reaktion entstehende Reaktionswärme abführen zu können.

Bevorzugterweise wird ein Schaufeltrockner mit einem Siedekühler ("Rückflußkühler") als Reaktor verwendet. Bei einer derartigen Kombination wird die im Reaktor entstehende Wärme durch Verdampfung des Lösungsmittels und/oder Produkts aus dem Reaktor abgeführt. Die Dämpfe werden an einem Wärmetauscher, dem Siedekühler, wieder kondensiert, und üblicherweise in den Reaktor zurückgeführt oder aufgearbeitet, beispielsweise durch Gewinnung des Produkts.

Die Maische wird mit der Verbindung der Formel R'-CHal₃ zur Reaktion gebracht. Dabei entstehen der Orthoester und das Alkalihalogenid. Der Orthoester wird gemeinsam mit dem in der Maische enthaltenen Alkohol vom Alkalihalogenid abgetrennt, beispielsweise durch Filtration oder Abdampfen, und anschließend wird der Orthoester vom Alkohol abdestilliert. Die Reaktion kann kontinuierlich oder diskontinuierlich durchgeführt werden.

In einer bevorzugten diskontinuierlichen Verfahrensweise wird die Maische in einem Schaufeltrockner hergestellt, indem festes Alkalialkoholat im Schaufeltrockner gerührt wird und die gewünschte Menge Alkohol eindosiert wird. Die freiwerdende Lösungswärme wird dabei über Siedekühlung abgeführt, die Dämpfe in einem Siedekühler kondensiert und das Kondensat in den Schaufeltrockner zurückgeführt. Üblicherweise dauert die Herstellung der Maische bei üblichen Baugrößen von Schaufeltrocknern, üblichen Siedekühlern und dadurch bestimmten Chargengrößen zwischen 5 Minuten und einer Stunde.

Anschließend wird die Maische mit der Verbindung der Formel R'-CHal₃ umgesetzt. Bei diskontinuierlichem Betrieb wird dieser Verfahrensschritt zweckmäßigerweise im gleichen Reaktor durchgeführt wie die Herstellung der Maische, prinzipiell können jedoch auch getrennte Reaktoren verwendet werden. Eine zwischenzeitliche Abkühlung der siedenden Maische ist nicht notwendig, kann aber erfolgen.

Die Verbindung der Formel R'-CHal₃ wird in die gerührte Maische unter Siedekühlung und Rückfluß des Kondensats eindosiert. Die Zudosierungsgeschwindigkeit wird im wesentlichen durch die Kühlleistung des Siedekühlers bestimmt. Die Zudosierung dauert bei üblichen Baugrößen von Schaufeltrocknern, üblichen Siedekühlern und dadurch bestimmten Chargengrößen in der Regel zwischen 30 Minuten und 2 Stunden, meist etwa eine Stunde. Vorzugsweise wird das Entstehen einer übermäßig hohen lokalen Konzentration der Verbindung der Formel R'-CHal₃ vermieden, dazu wird beispielsweise die Verbindung der Formel R'-CHal₃ entweder an mehreren über den Schaufeltrockner verteilten Stellen zudosiert oder in einer besonders bevorzugten Ausführungsform dem aus dem Kühler zurücklaufenden Kondensat zugemischt. Solche Maßnahmen zu Erniedrigung der lokalen Konzentration der Verbindung der Formel R'-CHal₃ können auch kombiniert werden. Das molare Verhältnis von eingesetztem Alkalialkoholat zu eingesetzter Verbindung der Formel R'-CHal₃ beträgt etwa 3 : 1. Bei Verwendung eines stöchiometrischen Unterschusses an Verbindung der Formel R'-CHal₃ bleibt das Reaktionsmedium stets alkalisch, was meist die Anforderungen an die Korrosionsfestigkeit von Stählen, die mit dem Reaktionsmedium in Kontakt kommen, absenkt und daher häufig die Verwendung preiswerterer Stähle als Anlagenwerkstoffe ermöglicht. Bei Verwendung eines stöchiometrischen Überschusses an Verbindung der Formel R'-CHal₃ verbleiben im entstehenden Nebenprodukt Alkalihalogenid nur sehr geringe Anteile an nicht abreagiertem Alkalialkoholat, was seine Entsorgung erleichtert. Ob ein stöchiometrischer Überschuß oder Unterschuß an Verbindung der Formel R'-CHal₃ verwendet wird oder ob dieses möglichst exakt stöchiometrisch eingesetzt wird, ist daher ein anhand der konkreten Gegebenheiten wie etwa den verwendeten Anlagenwerkstoffen, den vorhandenen technischen Möglichkeiten zur Abtrennung und Reinigung von unumgesetzten Ausgangsstoffen sowie den Möglichkeiten zur Entsorgung der Rückstände zu optimierender Parameter. Vorzugsweise werden auf 3 Mol Alkalialkoholat höchstens 1,1 Mol Verbindung der Formel R'-CHal₃ eingesetzt, in besonders bevorzugter Weise höchstens 0,99 Mol. Vorzugsweise werden auf 3 Mol Alkalialkoholat mindestens 0,95 Mol Verbindung der Formel CHal₃ eingesetzt, in besonders bevorzugter Weise mindestens 0,97 Mol.

Die Reaktion wird im allgemeinen bei Normaldruck unter der sich einstellenden Siedetemperatur der Reaktionsmischung durchgeführt. Es ist genauso möglich, die Reaktion unter Druck durchzuführen, beispielsweise bei einem Druck von höchstens 10 bar, vorzugsweise höchstens 5 bar, wobei sich die Siedetemperatur entsprechend erhöht. Anwendung von Druck ermöglicht in der Regel höhere Raumzeitausbeuten, da die Reaktionstemperatur und damit der Umsatz steigt. Zu hohe Reaktionstemperaturen können jedoch zu verringerten Selektivitäten führen, so daß der Reaktionsdruck letztendlich im Einzelfall nach wirtschaftlichen Erwägungen anhand der Kapazitäten der Synthese- und Aufarbeitungsteile der Anlage gewählt wird.

Nachdem die Verbindung der Formel R'-CHal₃ vollständig zugegeben wurde, läßt man die Reaktionsmischung vorzugsweise noch einige Zeit unter Siedekühlung nachreagieren. Die Nachreaktionsdauer liegt bei üblichen Chargengrößen zwischen 5 Minuten und 5 Stunden, vorzugsweise zwischen 15 Minuten und 90 Minuten.

Anschließend werden die flüchtigen Komponenten der Reaktionsmischung von den festen Komponenten abgetrennt. Vorzugsweise werden dazu die flüchtigen Komponenten abgedampft, indem das im Siedekühler anfallende Kondensat nicht in den Reaktor zurückgeleitet wird, sondern in einem weiteren Behälter gesammelt wird. In diesem Behälter kann auch Kondensat aus mehreren Reaktoren gesammelt werden. Das Abdampfen der flüchtigen Komponenten geschieht vorzugsweise bei Normaldruck oder Unterdruck. Zur vollständigen Entfernung der flüchtigen Komponenten aus dem Reaktor ist es vorteilhaft, dessen Innenwand gegen Ende des Abdampfens auf eine Temperatur von mindestens 20 °C oberhalb der Siedetemperatur der Reaktionsmischung, vorzugsweise mindestens 50 °C oberhalb dieser Temperatur aufzuheizen. Im allgemeinen ist es dazu ausreichend, die Reaktorinnenwand auf höchstens 150 °C oberhalb der Siedetemperatur der Reaktionsmischung aufzuheizen, bevorzugterweise wird eine Temperatur von nicht mehr als 100 °C oberhalb dieser Siedetemperatur eingestellt. Diese Temperatur wird dabei so lange gehalten, bis das als Nebenprodukt im Reaktor zurückbleibende Alkalihalogenid im wesentlichen trocken ist. Bei üblichen Chargengrößen genügt dazu meist eine Zeit im Bereich von einer Stunde bis 10 Stunden, in der Regel eine Zeit im Bereich von zwei Stunden bis 4 Stunden. Das getrocknete Salz wird anschließend aus dem Reaktor entfernt.

Das gesammelte Kondensat, das im wesentlichen den Orthoester und den zum Anmaischen verwendeten Alkohol enthält, wird anschließend in seine Bestandteile aufgetrennt. Vorzugsweise geschieht dies durch Destillation in bekannter Weise. Das Wertprodukt, der Orthoester, wird in marktüblicher Reinheit, beispielsweise in einer Reinheit von 99,6 % bis 99,9 Gew.-%, entnommen. Die Destillation wird dazu bequemerweise so durchgeführt, daß der als Leichtsiederfraktion am Kopf der Destillationskolonne abdestillierte Alkohol mindestens 0,5 Gew.-%, meist jedoch mindestens 3 Gew.-%, vorzugsweise mindestens 19 Gew.-%, und in besonders bevorzugter Weise mindestens 25 Gew.-% Orthoester enthält. Aus Wirtschaftlichkeitsgründen ist es sinnvoll, den Gehalt des abdestillierten Alkohols an Orthoester auf höchstens 40 Gew.-%, vorzugsweise höchstens 30 Gew.-% Orthoester zu begrenzen.

Die hauptsächlich den Alkohol enthaltende Leichtsiederfraktion wird vorzugsweise erneut zur Anmaischung von Alkalialkoholat verwendet. Zur Vermeidung der Aufpegelung von Nebenprodukten wird aus dieser Leichtsiederfraktion ein Anteil ausgeschleust und verworfen. Dieser Anteil liegt im allgemeinen bei mindestens 2 Gew.-%, vorzugsweise mindestens 5 Gew.-%, und im allgemeinen höchstens bei 20 Gew.-%, vorzugsweise höchstens 10 Gew.-%.

Dieses Verfahren kann ebenso vollkontinuierlich durchgeführt werden. Dazu ist es in der Regel vorteilhaft, die Herstellung der Maische und die Umsetzung der Maische mit Verbindung der Formel R'-CHal₃ in getrennten Reaktoren vorzunehmen.

### Beispiel

In einem mit einem Rückflußkühler ausgestatteten Schaufeltrockner von 300 l Inhalt wurden 108 kg festes Natriummethanolat vorgelegt. Bei laufendem Mischwerk wurde innerhalb von 20 Minuten 126 kg Methanol zugepumpt, wobei sich der Trocknerinhalt auf 82 °C erwärmte, absiedendes Methanol im Rückflußkühler kondensierte und in den Trockner zurücklief. Das Verhältnis von festem zu gelöstem Natriummethanolat in der Maische betrug rund 1. Anschließend wurden binnen 90 Minuten über zwei getrennte Dosierstellen insgesamt 77,1 kg Chloroform bei Normaldruck zudosiert, wobei die Reaktionswärme durch Siedekühlung abgeführt wurde. Das im Rückflußkühler entstehende Kondensat lief in den Schaufeltrockner zurück. Gegen Ende der Chloroformzugabe wurde der Trockner auf 120 °C aufgeheizt und die Mischung weitere 45 Minuten unter Rückfluß gehalten. Danach wurde das im Rückflußkühler anfallende Kondensat nicht mehr in den Trockner zurückgeführt, sondern in einen Sammelbehälter geleitet. Der Trockner wurde gegen Ende dieses Abdampfvorgangs auf 180 °C aufgeheizt, nach weiteren 90 Minuten war das entstandene Natriumchlorid soweit getrocknet, daß es ohne zu verklumpen aus dem Trockner abgelassen werden konnte.

Das im Sammelbehälter aufgefangene Kondensat enthielt 34 Gew.-% TMOF. Es wurde kontinuierlich in eine Destillationskolonne mit 30 theoretischen Trennstufen auf der Höhe der 16. theoretischen Stufe eingeleitet. Die Kolonne wurde bei Normaldruck betrieben. Bei einem Rücklaufverhältnis von 1,7 wurde über einen Seitenabzug oberhalb des Kolonnensumpfs gasförmiges TMOF abgezogen und kondensiert. Das TMOF-Kondensat wies eine Reinheit von mindestens 99,8 Gew.-% auf. Am Kopf der Kolonne wurde eine Leichtsiederfraktion gewonnen, die zu rund 90 Gew.-% aus Methanol, zu rund 5 Gew.-% aus TMOF, zu rund 1,5 Gew.-% aus Chloroform und zu rund 2 Gew.-% aus leichtsiedenden Nebenkomponenten bestand. Diese Leichtsiederfraktion wurde wiederum zum Anmaischen von Natriummethanolat in weiteren Chargen verwendet. Insgesamt wurden derart 6 Chargen nacheinander hergestellt. Die Reinheit des gewonnenen TMOFs betrug stets mindestens 99,8 Gew.-%. Die bei der letzten Charge gewonnene Leichtsiederfraktion bestand aus rund 90 Gew. -% Methanol, rund 6,5 Gew.-% TMOF, rund 1,5 Gew.-% Chloroform und rund 2 Gew.-% leichtsiedenden Nebenkomponenten. Pro Charge wurden rund 66 kg TMOF gewonnen, was einer Ausbeute von rund 93 Mol-%, bezogen auf eingesetztes Natriummethanolat, entspricht.

### Vergleichsbeispiel

In einem mit einem Rückflußkühler ausgestatteten Schaufeltrockner von 300 l Inhalt wurden 180 kg einer 30 Gew.-%igen Lösung von Natriummethanolat in Methanol vorgelegt. Der Füllstand im Reaktor war dadurch ebenso hoch wie bei der im vorstehenden Beispiel beschriebenen Vorgehensweise. Bei laufendem Mischwerk wurde die Lösung auf 80 °C erwärmt, wobei absiedendes Methanol im Rückflußkühler kondensierte und in den Trockner zurücklief. Anschließend wurden binnen 90 Minuten über zwei getrennte Dosierstellen insgesamt 39,0 kg Chloroform bei Normaldruck zudosiert, wobei die Reaktionswärme durch Siedekühlung abgeführt wurde. Das im Rückflußkühler entstehende Kondensat lief in den Schaufeltrockner zurück. Nach dem Ende der Chloroformzugabe wurde die Mischung weitere 45 Minuten unter Rückfluß gehalten. Danach wurde das im Rückflußkühler anfallende Kondensat nicht mehr in den Trockner zurückgeführt, sondern in einen Sammelbehälter geleitet. Der Trockner wurde gegen Ende dieses Abdampfvorgangs auf 180 °C aufgeheizt, nach weiteren 90 Minuten war das entstandene Natriumchlorid soweit getrocknet, daß es ohne zu verklumpen aus dem Trockner abgelassen werden konnte.

Das im Sammelbehälter aufgefangene Kondensat enthielt 19,8 Gew. -% TMOF. Es wurde kontinuierlich in eine Destillationskolonne mit 70 theoretischen Trennstufen auf der Höhe der 36. theoretischen Stufe eingeleitet. Die Kolonne wurde bei Normaldruck betrieben. Bei einem Rücklaufverhältnis von 8 wurde über einen Seitenabzug im Abtriebsteil der Kolonne gasförmiges TMOF abgezogen und kondensiert. Das TMOF-Kondensat wies eine Reinheit von mindestens 99,7 Gew.-% auf. Am Kopf der Kolonne wurde eine Leichtsiederfraktion gewonnen. Diese Leichtsiederfraktion wurde zur Abtrennung des Methanols von sonstigen leichtsiedenden Komponenten in eine weitere Kolonne mit 40 theoretischen Trennstufen auf der Höhe der 35. theoretischen Stufe eingeleitet. Die Kolonne wurde bei Normaldruck betrieben. Bei einem Rücklaufverhältnis von 80 wurde am Sumpf Methanol, das unter 1 ppm Chloroform und unter 1000 ppm TMOF enthielt, abgezogen. Über Kopf wurde eine Fraktion, bestehend aus rund 60 Gew.-% Methanol und 40 Gew.-% sonstigen leichtsiedenden Komponenten, gewonnen. Insgesamt wurden 33,9 kg TMOF gewonnen, was einer Ausbeute von rund 96 Mol-%, bezogen auf eingesetztes Natriummethanolat, entspricht.

Das Beispiel und das Vergleichsbeispiel zeigen, daß das erfindungsgemäße Verfahren eine Ausbeute ermöglicht, die der beim technisch gängigen Verfahren entspricht, jedoch bei ungleich geringerem Trenn- und damit Energieaufwand und in wesentlich höherer Raumzeitausbeute.

## Patentansprüche

1. Verfahren zur Herstellung eines Orthoesters der Formel R'-C(OR)₃, wobei R und R' jeweils einen organischen Rest und R' auch Wasserstoff darstellen und wobei die Reste R im Orthoester gleich oder voneinander verschieden sind, durch Umsetzung einer Verbindung der Formel R'-CHal₃, wobei Hal Halogen darstellt, mit einem Alkalialkoholat der Formel ROM, wobei M Alkali darstellt, in Gegenwart des entsprechenden Alkohols der Formel ROH, oder im Falle voneinander verschiedener Reste R im Orthoester mit einem entsprechenden Gemisch von Alkalialkoholaten in einem entsprechenden Gemisch von Alkoholen, **dadurch gekennzeichnet, daß** man eine Maische des Alkalialkoholats oder der Alkalialkoholate im entsprechenden Alkohol oder Gemisch von Alkoholen einsetzt.

2. Verfahren nach Anspruch 1, **dadurch gekennzeichnet, daß** man eine Maische von Natriummethanolat in Methanol einsetzt.

3. Verfahren nach Anspruch 1, **dadurch gekennzeichnet, daß** man eine Maische von Natriumethanolat in Ethanol einsetzt.

4. Verfahren nach Anspruch 1, **dadurch gekennzeichnet, daß** man eine Maische mit einem Gewichtsverhältnis von festem zu gelöstem Alkalialkoholat von mindestens 0,4 verwendet.

5. Verfahren nach Anspruch 4, **dadurch gekennzeichnet, daß** man eine Maische mit einem Gewichtsverhältnis von festem zu gelöstem Alkalialkoholat von höchstens 3 verwendet.

6. Verfahren nach Anspruch 1, **dadurch gekennzeichnet, daß** man auf 3 Mol Alkalialkoholat höchstens 1,1 Mol Verbindung der Formel R'-CHal₃ einsetzt.

7. Verfahren nach Anspruch 1, **dadurch gekennzeichnet, daß** man die Umsetzung unter einem Druck von mindestens Normaldruck und höchstens 10 bar Überdruck durchführt.

8. Verfahren nach Anspruch 1, **dadurch gekennzeichnet, daß** man die Isolierung des Produkts durch Destillation durchführt und dabei eine im wesentlichen den Alkohol enthaltende Fraktion gewinnt, die darüber hinaus zwischen 0,5 und 40 Gew.-% Orthoester enthält.

9. Verfahren nach Anspruch 8, **dadurch gekennzeichnet, daß** man die im wesentlichen den Alkohol enthaltende Fraktion zum Anmaischen von Alkalialkoholat verwendet.

## Claims

1. Process for preparing an orthoester of the formula R'-C(OR)₃, where R and R' are each an organic radical and R' is also hydrogen and where the radicals R in the orthoester are identical or different from one another, by reacting a compound of the formula R'-CHal₃, where Hal is halogen, with an alkali metal alkoxide of the formula ROM, where M is alkali metal, in the presence of the corresponding alcohol of the formula ROH, or, in the case of radicals R in the orthoester which differ from one another, with a corresponding mixture of alkali metal alkoxides in a corresponding mixture of alcohols, which comprises using a slurry of the alkali metal alkoxide or the alkali metal alkoxides in the corresponding alcohol or mixture of alcohols.

2. A process as claimed in claim 1, wherein a slurry of sodium methoxide in methanol is used.

3. A process as claimed in claim 1, wherein a slurry of sodium ethoxide in ethanol is used.

4. A process as claimed in claim 1, wherein use is made of a slurry having a weight ratio of solid to dissolved alkali metal alkoxide of at least 0.4.

5. A process as claimed in claim 4, wherein use is made of a slurry having a weight ratio of solid to dissolved alkali metal alkoxide of at most 3.

6. A process as claimed in claim 1, wherein at most 1.1 mol of compound of the formula R'-CHal₃ is used per 3 mol of alkali metal alkoxide.

7. A process as claimed in claim 1, wherein the reaction is carried out under at least atmospheric pressure and at most 10 bar superatmospheric pressure.

8. A process as claimed in claim 1, wherein the product is isolated by distillation which produces an essentially alcohol-containing fraction which, in addition, comprises from 0.5 to 40% by weight of orthoesters.

9. A process as claimed in claim 8, wherein use is made of the essentially alcohol-containing fraction for slurrying alkali metal alkoxide.

## Revendications

1. Procédé de préparation d'un ortho-ester de formule R'-C(OR)₃, dans laquelle R et R' représentent chacun un résidu organique et R' également un atome d'hydrogène, et dans laquelle les résidus R dans l'ortho ester sont identiques ou différents les uns des autres, par réaction d'un composé de formule R'-CHal₃, dans laquelle Hal représente un atome d'halogène, avec un alcoolate de métal alcalin de formule ROM, dans laquelle M représente un métal alcalin, en présence de l'alcool correspondant de formule ROH, ou bien dans le cas de résidus R différents les uns des autres dans l'ortho-ester avec un mélange correspondant d'alcoolates de métal alcalin dans un mélange correspondant d'alcools, **caractérisé en ce que** l'on met en oeuvre un moût de l'alcoolate de métal alcalin, ou des alcoolates de métal alcalin, dans l'alcool ou le mélange d'alcools correspondants.

2. Procédé selon la revendication 1, **caractérisé en ce que** l'on met en oeuvre un moût de méthanolate de sodium dans du méthanol.

3. Procédé selon la revendication 1, **caractérisé en ce que** l'on met en oeuvre un moût d' éthanolate de sodium dans de l'éthanol.

4. Procédé selon la revendication 1, **caractérisé en ce que** l'on emploie un moût ayant un rapport pondéral de l'alcoolate de métal alcalin solide à celui dissous, d'au moins 0,4.

5. Procédé selon la revendication 4, **caractérisé en ce que** l'on emploie un moût ayant un rapport pondéral de l'alcoolate de métal alcalin solide à celui dissous, de 3 au maximum.

6. Procédé selon la revendication 1, **caractérisé en ce que** l'on met en oeuvre au maximum 1,1 mole de composé de formule R' -CHal₃, sur 3 moles d'alcoolate de métal alcalin.

7. Procédé selon la revendication 1, **caractérisé en ce que** la réaction est réalisée sous une pression d'au moins la pression normale et une surpression de 10 bars au maximum.

8. Procédé selon la revendication 1, **caractérisé en ce que** l'on réalise l'isolation du produit par distillation et qu'en même temps l'on extrait la fraction contenant essentiellement l'alcool, qui, de surcroît, contient entre 0,5 et 40% en poids d'ortho-ester.

9. Procédé selon la revendication 8, **caractérisé en ce que** l'on emploie la fraction contenant essentiellement l'alcool pour le malaxage de l'alcoolate de métal alcalin.
